# EUROPEAN PATENT APPLICATION

(11) **EP 2 425 781 A1**
(43) Date of publication of application: **07.03.2012**
(21) Application number: 10769365.7
(22) Date of filing: 26.04.2010
(51) Int. Cl.: A61B 6/04

(54) **THERMOPLASTIC MASK SECURING FRAME**

(30) Priority: 29.04.2009 ES 200901117
(71) Applicant: Fundación Andaluza Beturia Para La Investigación En Salud (FABIS), 21005 Huelva (ES)
(72) Inventor: VELÁZQUEZ, Santiago, E-21005 Huelva (ES)
(74) Representative: Pons Ariño, Angel
(86) International application number: PCT/ES2010/070263
(87) International publication number: WO 2010/125219

(57) **Abstract**

The present invention describes a frame (1) for securing a thermoplastic mask (2) suitable for the portable coil of a nuclear magnetic resonance apparatus and for a computerized tomography apparatus, which comprises a first support portion (1a) for the upper part of the patient's back and a second support portion (1b) for securing the patient's head in an upright position, the two support portions (1a, 1b) comprising means (3a, 3b) for anchoring the thermoplastic mask (2) and the anchoring means (3b) of the second support portion (1b) securing the thermoplastic mask (2) behind the patient's head.

## Description

### OBJECT OF THE INVENTION

The present invention is directed to a thermoplastic mask attachment frame, specifically for the immobilization of the head of a patient, which is suitable for both its use in the portable coil of nuclear magnetic resonance (NMR) machines and in a computerized tomography (CT) machine.

### BACKGROUND OF THE INVENTION

Currently, there are a large number of devices whose object is to immobilize a part of the body of a patient, for example during the taking of radiological images or during radiation treatments. In the specific case of the head, these devices can be formed by a frame on which a mesh of thermoplastic material that adapts to the shape of the head of the patient is fixed. However, the immobilization devices used in computerized tomography (CT) machines are not suitable for their use in the portable coil of nuclear magnetic resonance (NMR) machines, since the room in said portable coil is very small. This fact causes these devices to be used only in CT to immobilize the head of the patient in the habitual position in radiation therapy, i.e., with the patient facing front. On the contrary, in NMR the head of the patient is not immobilized with precision, using in addition a position contrary to that of treatment, i.e. with hyperextension of the neck, or in other words, as if the patient was looking upwards. The use of different positions in CT and NMR, in turn, causes the fusion of images obtained by means of these two techniques to be extremely complex. The possibilities of movement of the cervical vertebrae make it impossible to assure the necessary level quality of anatomical correlation of images at all levels, even with the most sophisticated image fusion algorithms.

### DESCRIPTION OF THE INVENTION

The present invention describes a thermoplastic mask attachment frame for magnetic resonance and computerized tomography that solves the problems described. Thanks to the portion of support of the head being narrower than usual and the anchor means of the mask to the frame being significantly less bulky than the existing ones, the frame of the invention allows its insertion in the portable coil of a nuclear magnetic resonance machine.

In the present document, we will call "upright" position to the position normally used in radiation therapy and CT machines, in which the patient is facing front. On the contrary, the usual position in the portable coils of NMR machines up to now, where the patient is looking up will be called "hyperextended".

According to a first aspect of the present invention, a thermoplastic mask attachment frame suitable for nuclear magnetic resonance (NMR) and computerized tomography (CT) is described, which is formed by a first support portion for the upper part of the back of the patient and a second support portion to hold the head of the patient in an upright position. Besides, both support portions comprise attaching means for attaching the thermoplastic mask, being the attaching means of the second support portion arranged in such way that they hold the patient's head from behind. In the present context, this statement refers not only to the area located behind the patient's head itself, but also his neck (in general, the entire skull), in opposition to the current immobilization devices, where the thermoplastic mask is fixed to the frame on the sides of the patient's head, perpendicularly to the frame. Thus, upon changing the area where the thermoplastic mask is attached, the patient's head is better immobilized, especially against lateral movements.

Besides, this change in the area where the thermoplastic mask is attached allows diminishing the width of the second portion of support, which in turn, allows its introduction in the portable coil of a nuclear magnetic resonance machine. Thus, it is possible to sequentially take images of the patient's head by means of CT and NMR without modifying the position of said patient's head, which will be a natural upright position at all times. In preferred embodiments of the invention, the width of this second portion of support is less than 20 cm, more preferably less than 17 cm. With regard to its length, it will preferably be between 25 cm. and 30 cm.

The attachment means of the thermoplastic mask can be of any kind as long as it achieves an adequate attachment and take up a sufficiently reduced room as to allow the introduction of the attachment frame with the thermoplastic mask in the portable coil of a NMR machine. Nevertheless, according to a particular embodiment of the invention, the attaching means of the thermoplastic mask have the shape of grooves arranged on the sides or the edge of the frame, in the areas of both the first and the second support portions, which allow the introduction of some edges of the thermoplastic mask, approximately like in a "dovetail" mechanism.

In another preferred embodiment of the invention, the second support portion comprises attaching means for attaching a headrest with the purpose of improving the patient's comfort, besides positioning his head with greater precision. For example, the attaching means can comprise two through holes located in the longitudinal axis of the second support portion, although other configurations would be possible. The second support portion can also comprise some tholepins for shaping the headrest.

The material that forms the frame of the invention must be radiotransparent and non conductor so as not to alter the images of NMR. For example, it could be a central layer of expanded PVC covered with vinyl and nuts and bolts made of nylon.

Finally, a second aspect of the present invention is directed to the use of the frame described above for taking sequential images in the portable coil of a nuclear magnetic resonance (NMR) machine and in a computerized tomography (CT) machine. That is to say, images of the patient in different positions in each of these machines would not be taken anymore, but rather the frame of the invention could be used for both. It is understood in this case that the term "sequential" is not restrictive of the order in which the CT and NMR are used.

### SHORT DESCRIPTION OF THE DRAWINGS

- Fig.1: shows a perspective view of an embodiment of the frame of the invention.
- Fig.2: shows a view in plant of the framework of Fig. 1

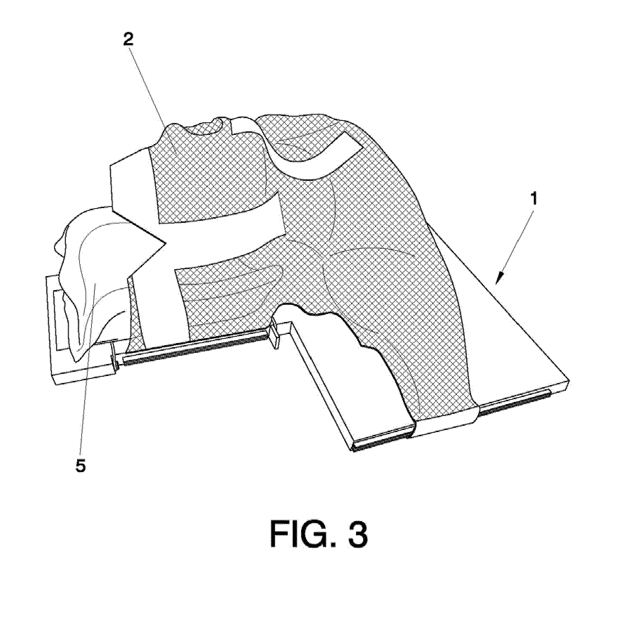

- Fig.3: shows a perspective view of the frame of Figs. 1 and 2 with thermoplastic mask and headrest.

### PREFERRED EMBODIMENT OF THE INVENTION

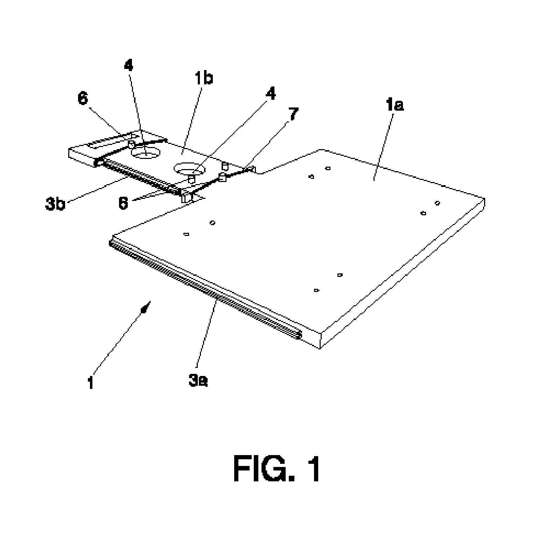

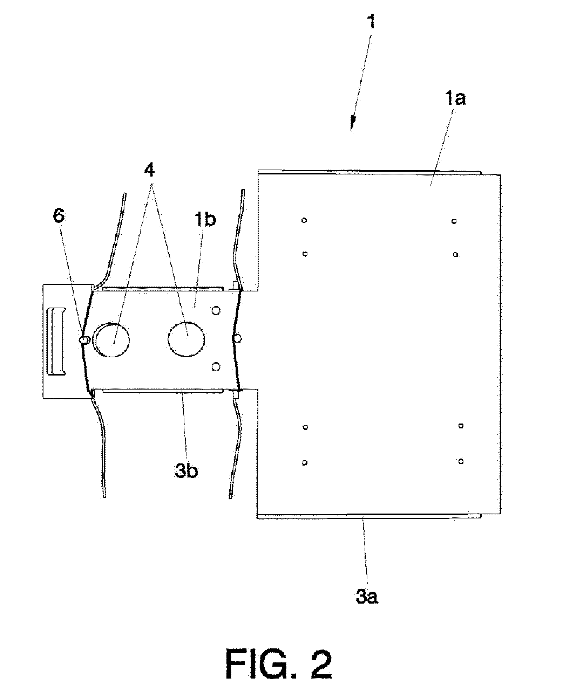

Next an embodiment of a thermoplastic mask (2) attachment frame (1) is described, referring to the appended figures. In Figs. 1 and 2 a perspective and a plant view of the frame (1) are shown respectively, wherein the first portion (1a) and the second portion (1b), which in this example are made of a single piece, can be seen. The first and second attaching means (3a, 3b), which in this example are two grooves of the "dovetail" type, are also shown. In addition, the second portion (1b) has attaching means (4) for the headrest (5), which in this example are a pair of holes in its longitudinal axis, and some small tholepins (6) that help shape the headrest (5) in the area of the neck of the patient.

The figures also show some elastic bands (7) arranged around the second portion (1b) of the frame (1) and which serve to provide an additional fastening to the thermoplastic mask. Finally, Fig, 3 shows the frame (1) of the invention to which the thermoplastic mask (2) and the headrest (5) have already been attached.

## Claims

1. Thermoplastic mask (2) attachment frame (1) suitable for the portable coil of a nuclear magnetic resonance (NMR) machine and for a computerized tomography (CT) machine, **characterized in that** it comprises a first support portion of support (1a) for the upper part of the patient's back and a second support portion (1b) to hold the patient's head in an upright position, the first and the second support portions (1 a, 1b) comprising first and second attaching means (3a, 3b) for attaching the thermoplastic mask (2), and wherein the second attaching means (3b) of the thermoplastic mask (2) hold said mask (2) behind the patient's head.

2. Frame (1) according to claim 1, wherein the width of the second support portion (1b) is less than 20 cm.

3. Frame (1) according to claim 2, wherein the width of the second support portion (1b) is less than 17 cm.

4. Frame (1) according to any of the previous claims, wherein the length of the second support portion (1b) is between 25 cm and 30 cm.

5. Frame (1) according to any of the previous claims, wherein the first and second attaching means (3a,3b) of the thermoplastic mask (2) are grooves arranged on the sides of the first and second support portions (1a, 1b).

6. Frame (1) according to any of the previous claims, wherein the second support portion (1b) comprises attaching means (4) for attaching a headrest (5).

7. Frame (1) according to claim 6, wherein the attaching means (4) for the attachment of the headrest (5) comprises two through holes located in the longitudinal axis of said second support portion (1b).

8. Frame (1) according to any of claims 6-7, wherein the second support portion (1b) also comprises some tholepins (6) for shaping the headrest (5).

9. Sequential use of a frame (1) according to any of the previous claims in the portable coil of a nuclear magnetic resonance (NMR) machine and in a computerized tomography (CT) machine.
